# EUROPEAN PATENT APPLICATION

(11) **EP 1 776 982 A1**
(43) Date of publication of application: **25.04.2007**
(21) Application number: 05022665.3
(22) Date of filing: 18.10.2005
(51) Int. Cl.: A61P 29/00, A61K 31/519, C07D 491/04

(54) **Pyrimidine compounds as histamine modulators**

(71) Applicant: Argenta Discovery Limited, Harlow, Essex CM19 5TR (GB)
(72) Inventor: Harris, Neil, Flex Meadow Harlow, Essex, CM19 5TR (GB); Higgs, Christopher, Flex Meadow Harlow, Essex,CM19 5TR (GB); Wren, Stephen, Flex Meadow Harlow, Essex, CM19 5TR (GB); Dyke, Hazel, Flex Meadow Harlow, Essex, CM19 5TR (GB); Price, Steve, Flex Meadow Harlow, Essex, CM19 5TR (GB); Cramp, Sue, Flex Meadow Harlow, Essex, CM19 5TR (GB)
(74) Representative: Huhn, Michael

(57) **Abstract**

The present invention relates to novel pyrimidine compounds, for the modulation of the histamine H4 receptor and the treatment or prevention of conditions mediated by the histamine H4 receptor. The invention also relates to the preparation of such compounds.

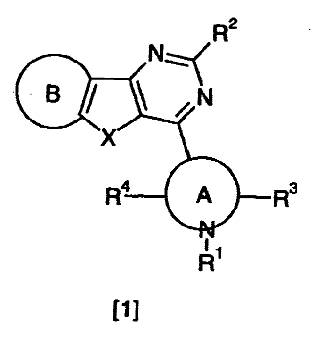

## Description

### Field of the Invention

The invention relates to novel pharmaceutically active fused heterocyclic compounds and methods of using them to treat or prevent diseases mediated by the histamine H4 receptor alone or by the histamine H1 and H4 receptors in combination.

### Background of the Invention

Histamine exerts its various physiological functions through interactions with four receptors of the G-protein coupled superfamily (the histamine H1, H2, H3, and H4 receptors). Compounds that antagonise the effects of histamine at the H1 and H2 receptors have found utility in the treatment of a number of different diseases. For example, histamine H1 receptor antagonists have beneficial effects in the treatment of some allergies, and H2 receptor antagonists have valuable effects in the treatment of gastric ulcers. Compounds that antagonise the H3 receptor may also have beneficial effects, for example in treating diseases such as attention deficit hyperactivity disorder, insomnia, and eating disorders. The recent discovery of the histamine H4 receptor (Nakamura et al, Biochem. Biophys. Res. Commun., 2000, 279, 615-620) has led to efforts to determine whether compounds that modulate the effects of this receptor may also have useful properties.

Expression profiling for the H4 receptor indicates that it is highly expressed in peripheral tissues that are implicated in inflammatory responses, such as leucocytes, spleen, lung, and liver (Coge et al, Biochem. Biophys. Res. Commun., 2001, 284, 301-309; Oda et al, J. Biol. Chem., 2000, 275, 36781-36786). Further evidence has been obtained that the H4 receptor may play a role in inflammatory diseases, in particular asthma and other allergic diseases. For example, the H4 receptor has been shown to play a role in eosinophil chemotaxis and shape change (O'Reilly et al, J. Recept. Signal Trans. Res., 2002, 22, 431-448; Buckland et al, Br. J. Pharmacol., 2003, 140, 1117-1127; Ling et al, Br. J. Pharmacol., 2004, 142, 161-171). Similarly, histamine has been shown to mediate the signalling and chemotaxis of mast cells *via* the H4 receptor (Hosftra et al, J. Pharmacol. Exp. Ther., 2003, 305, 1212-1221). Therefore, compounds that antagonise the effects of the H4 receptor may have utility in the prevention and treatment of a number of diseases, including inflammatory conditions mediated by leucocytes and mast cells.

The present invention is based in part on the teachings of WO2003057919 and WO2004021999, in which the use of histamine H4 receptor modulators for the prevention, treatment, induction, or other desired modulation of inflammatory responses, inflammation, or diseases and/or conditions that are modulated, affected, or caused by inflammation or inflammatory responses, is described. The present invention is also based in part on the teachings of WO2002072548, US2003207893, US2004048878, WO2004022060, and WO2004022061, which disclose novel compounds that are useful for the treatment of H4-mediated diseases.

The use of a histamine H1 receptor antagonist for the treatment of allergic rhinitis is well understood. Publication WO2002056871 teaches that the combination of a selective H4 receptor antagonist with a H1 receptor antagonist may have utility for the treatment of a range of diseases that are modulated by either or both of the H1 and H4 receptors. Similarly, WO2004066960 teaches that the administration of one or more histamine H3 receptor antagonists, one or more histamine H4 receptor antagonists, and, optionally, one or more histamine H1 antagonists, may have utility in the treatment or prevention of conditions characterised by airway inflammation. Neither of these documents describe the potential utilities of compounds that combine the properties of H1 and H4 receptor antagonism in one molecular entity.

We have now surprisingly found that pyrimidine compounds of general structure [1] represent a novel class of histamine modulators that antagonise the effects of the histamine H4 receptor and, optionally, the H1 receptor.

### Summary of the invention

One aspect of the invention provides compounds of general formula [1]: 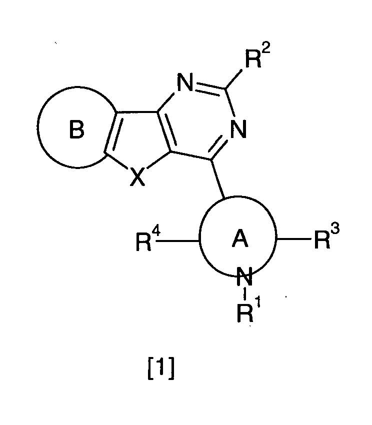 in which:
A represents a fully saturated or partially unsaturated ring of 5 to 7 atoms, at least one of which is a nitrogen atom;
B represents aryl or heteroaryl ring of 5 to 6 atoms, wherein B is optionally substituted with one up to three groups of formula R⁵, where R⁵ represents independently: H, F, Cl, Br, I, C₁₋₄-alkyl, C₃₋₆-cycloalkyl, heterocycloalkyl, C₁₋₄-alkoxy, C₃₋₆-cycloalkoxy, OH, OCF₃, CF₃, cyano, or NR⁶R⁷; R⁶ and R⁷ being independently H or C₁₋₄-alkyl;
X represents O, NH, S, or CH₂;
R¹ represents H, or C₁₋₄-alkyl;
R² represents H, optionally substituted C₁₋₄-alkyl, optionally substituted C₃₋₆-cycloalkyl, or optionally substituted aryl or heteroaryl;
R³ and R⁴ represent independently H, or C₁₋₂-alkyl; or R³ and R⁴ taken together may represent a C₁₋₄-alkylene group;
and corresponding *N*-oxides, pharmaceutically acceptable salts, solvates and prodrugs of such compounds.

A second aspect of the invention is a pharmaceutical composition comprising a compound of formula [1] or an *N*-oxide, pharmaceutically acceptable salt, solvate or prodrug thereof, in admixture with a pharmaceutically acceptable carrier or excipient.

A third aspect of the invention is a compound of formula [1] or an *N*-oxide, pharmaceutically acceptable salt, solvate or prodrug thereof for use in therapy and for use as a medicament, respectively.

A fourth aspect of the invention is the use of a compound of formula [1], or an *N*-oxide, pharmaceutically acceptable salt, solvate or prodrug thereof, in the manufacture of a medicament for the treatment of a disease in which a selective histamine H4 receptor antagonist or a mixed histamine H4 and H1 antagonist can prevent, inhibit or ameliorate the pathology and/or symptomatology of the disease.

A fifth aspect of the invention is a method for treating a disease in a patient in which a selective histamine H4 receptor antagonist or a mixed histamine H4 and H1 antagonist can prevent, inhibit or ameliorate the pathology and / or symptomatology of the disease, which method comprises administering to the patient a therapeutically effective amount of compound of formula [1] or an *N*-oxide, pharmaceutically acceptable salt, solvate or prodrug thereof.

A sixth aspect of the invention is a method of preparing a compound of formula [1] or an *N*-oxide, pharmaceutically acceptable salt, solvate or prodrug thereof.

A seventh aspect of the invention is a method of making a pharmaceutical composition comprising combining a compound of formula [1], or an *N*-oxide, pharmaceutically acceptable salt, solvate or prodrug thereof, with a pharmaceutically acceptable carrier or excipient.

For purposes of the present invention, the following definitions as used throughout the description of the invention shall be understood to have the following meanings:
"Compounds of the invention", and equivalent expressions, are meant to embrace compounds of general formula [1] as hereinbefore described, their *N*-oxides, their prodrugs, their pharmaceutically acceptable salts and their solvates, where the context so permits.
"Patient" includes both human and other mammals.
"Selective" and "selectivity", in the context of biological assays, refer to the ratio between responses in comparable H1 and H4 assays. Typically, for example, a selective compound might have a ratio between Kᵢ values for the H1 and H4 receptor binding assays of ≥ 100. Compounds of the invention that have selectivity ratios of < 100 are considered to be non-selective or mixed H1 and H4 antagonists.
"Antagonism" and "antagonist", in the context of H1 and H4 functional biological assays, refer to compounds of the invention that reduce the biological response produced by the application of an agonist (e.g. histamine) to either or both receptors, or reduce the constitutive biological response produced by either or both receptors in the absence of an agonist. Therefore, the terms antagonism and antagonists are also taken to include "partial agonism" and "partial agonist", and "inverse agonism" and "inverse agonist".

For purposes of the present invention, the following chemical terms as used above, and throughout the description of the invention, and unless otherwise indicated, shall be understood to have the following meanings:
"Acyl" means a -CO-alkyl group in which the alkyl group is as described herein. Exemplary acyl groups include -COCH₃ and -COCH(CH₃)₂.
"Acylamino" means a -NR-acyl group in which R and acyl are as described herein. Exemplary acylamino groups include -NHCOCH₃ and -N(CH₃)COCH₃.
"Alkoxy" and "alkyloxy" means an -O-alkyl group in which alkyl is as defined below. Exemplary alkoxy groups include methoxy and ethoxy. C₁₋₄-alkoxy means O-C₁₋₄-alkyl, respectively.
"Alkoxycarbonyl" means a -COO-alkyl group in which alkyl is as defined below. Exemplary alkoxycarbonyl groups include methoxycarbonyl and ethoxycarbonyl.
"Alkyl" as a group or part of a group refers to a straight or branched chain saturated hydrocarbon group having from 1 to 12, preferably 1 to 6, more preferred 1 to 4 (C₁₋₄-alkyl), carbon atoms, in the chain. Exemplary alkyl groups include methyl, ethyl, 1-propyl and 2-propyl.
"Alkenyl" as a group or part of a group refers to a straight or branched chain hydrocarbon group having from 1 to 12, preferably 1 to 6, carbon atoms and one carbon-carbon double bond in the chain. Exemplary alkenyl groups include ethenyl, 1-propenyl, and 2-propenyl.
"Alkylamino" means a -NH-alkyl group in which alkyl is as defined above. Exemplary alkylamino groups include methylamino and ethylamino.
"Alkylene means an -alkyl- group in which alkyl is as defined previously. Exemplary alkylene groups include -CH₂-, -(CH₂)₂- and -C(CH₃)HCH₂-.
"Alkenylene" means an -alkenyl- group in which alkenyl is as defined previously. Exemplary alkenylene groups include -CH=CH-, -CH=CHCH₂-, and -CH₂CH=CH-.
"Alkylsufinyl" means a -SO-alkyl group in which alkyl is as defined above. Exemplary alkylsulfinyl groups include methylsulfinyl and ethylsulfinyl.
"Alkylsufonyl" means a -SO₂-alkyl group in which alkyl is as defined above. Exemplary alkylsulfonyl groups include methylsulfonyl and ethylsulfonyl.
"Alkylthio" means a -S-alkyl group in which alkyl is as defined above. Exemplary alkylthio groups include methylthio and ethylthio.
"Aminoacyl" means a -CO-NRR group in which R is as herein described. Exemplary aminoacyl groups include -CONH₂ and -CONHCH₃.
"Aminoalkyl" means an alkyl-NH₂ group in which alkyl is as previously described. Exemplary aminoalkyl groups include -CH₂NH₂.
"Aminosulfonyl" means a -SO₂-NRR group in which R is as herein described. Exemplary aminosulfonyl groups include -SO₂NH₂ and -SO₂NHCH₃.
"Aryl" as a group or part of a group denotes an optionally substituted monocyclic or multicyclic aromatic carbocyclic moiety of from 6 to 14 carbon atoms, preferably from 6 to 10 carbon atoms, such as phenyl or naphthyl, and in one embodiment preferably phenyl. The aryl group may be substituted by one or more substituent groups.
"Arylalkyl" means an aryl-alkyl- group in which the aryl and alkyl moieties are as previously described. Preferred arylalkyl groups contain a C₁₋₄-alkyl moiety. Exemplary arylalkyl groups include benzyl, phenethyl and naphthlenemethyl.
"Arylalkyloxy" means an aryl-alkyloxy- group in which the aryl and alkyloxy moieties are as previously described. Preferred arylalkyloxy groups contain a C₁₋₄-alkyl moiety. Exemplary arylalkyl groups include benzyloxy.
"Aryl-fused-cycloalkyl" means a monocyclic aryl ring, such as phenyl, fused to a cycloalkyl group, in which the aryl and cycloalkyl are as described herein. Exemplary aryl-fused-cycloalkyl groups include tetrahydronaphthyl and indanyl. The aryl and cycloalkyl rings may each be sustitued by one or more substituent groups. The aryl-fused-cycloalkyl group may be attached to the remainder of the compound of formula [1] by any available carbon atom.
"Aryl-fused-heterocycloalkyl" means a monocyclic aryl ring, such as phenyl, fused to a heterocycloalkyl group, in which the aryl and heterocycloalkyl are as described herein. Exemplary aryl-fused-heterocycloalkyl groups include tetrahydroquinolinyl, indolinyl, benzodioxinyl, benxodioxolyl, dihydrobenzofuranyl and isoindolonyl. The aryl and heterocycloalkyl rings may each be sustitued by one or more substituent groups. The aryl-fused-heterocycloalkyl group may be attached to the remainder of the compound of formula [1] by any available carbon or nitrogen atom.
"Aryloxy" means an -O-aryl group in which aryl is described above. Exemplary aryloxy groups include phenoxy.
"Cyclic amine" means an optionally substituted 3 to 8 membered monocyclic cycloalkyl ring system where one of the ring carbon atoms is replaced by nitrogen, and which may optionally contain an additional heteroatom selected from O, S or NR (where R is as described herein). Exemplary cyclic amines include pyrrolidine, piperidine, morpholine, piperazine and *N*-methylpiperazine. The cyclic amine group may be substituted by one or more substituent groups.
"Cycloalkyl" means an optionally substituted saturated monocyclic or bicyclic ring system of from 3 to 12 carbon atoms, preferably from 3 to 8 carbon atoms, and more preferably from 3 to 6 carbon atoms (C₃₋₆-cycloalkyl). Exemplary monocyclic cycloalkyl rings include cyclopropyl, cyclopentyl, cyclohexyl and cycloheptyl. The cycloalkyl group may be substituted by one or more substituent groups. "Cycloalkoxy" means Cycloalkyl-O, respectively.
"Cycloalkylalkyl" means a cycloalkyl-alkyl- group in which the cycloalkyl and alkyl moieties are as previously described. Exemplary monocyclic cycloalkylalkyl groups include cyclopropylmethyl, cyclopentylmethyl, cyclohexylmethyl and cycloheptylmethyl.
"Dialkylamino" means a -N(alkyl)₂ group in which alkyl is as defined above. Exemplary dialkylamino groups include dimethylamino and diethylamino.
"Halo" or "halogen" means fluoro, chloro, bromo, or iodo. Preferred are fluoro or chloro.
"Haloalkoxy" means an -O-alkyl group in which the alkyl is substituted by one or more halogen atoms. Exemplary haloalkyl groups include trifluoromethoxy and difluoromethoxy.
"Haloalkyl" means an alkyl group which is substituted by one or more halo atoms. Exemplary haloalkyl groups include trifluoromethyl.
"Heteroaryl" as a group or part of a group denotes an optionally substituted aromatic monocyclic or multicyclic organic moiety of from 5 to 14 ring atoms, preferably from 5 to 10 ring atoms, in which one or more of the ring atoms is/are element(s) other than carbon, for example nitrogen, oxygen or sulfur. Examples of such groups include benzimidazolyl, benzoxazolyl, benzothiazolyl, benzofuranyl, benzothienyl, furyl, imidazolyl, indolyl, indolizinyl, isoxazolyl, isoquinolinyl, isothiazolyl, oxazolyl, oxadiazolyl, pyrazinyl, pyridazinyl, pyrazolyl, pyridyl, pyrimidinyl, pyrrolyl, quinazolinyl, quinolinyl, tetrazolyl, 1,3,4-thiadiazolyl, thiazolyl, thienyl and triazolyl groups. The heteroaryl group may be substituted by one or more substituent groups. The heteroaryl group may be attached to the remainder of the compound of formula [1] by any available carbon or nitrogen atom.
"Heteroarylalkyl" means a heteroaryl-alkyl- group in which the heteroaryl and alkyl moieties are as previously described. Preferred heteroarylalkyl groups contain a lower alkyl moiety. Exemplary heteroarylalkyl groups include pyridylmethyl.
"Heteroarylalkyloxy" means a heteroaryl-alkyloxy- group in which the heteroaryl and alkyloxy moieties are as previously described. Preferred heteroarylalkyloxy groups contain a lower alkyl moiety. Exemplary heteroarylalkyloxy groups include pyridylmethyloxy.
"Heteroaryloxy" means a heteroaryloxy- group in which the heteroaryl is as previously described. Exemplary heteroaryloxy groups include pyridyloxy.
"Heteroaryl-fused-cycloalkyl" means a monocyclic heteroaryl group, such as pyridyl or furanyl, fused to a cycloalkyl group, in which heteroaryl and cycloalkyl are as previously described. Exemplary heteroaryl-fused-cycloalkyl groups include tetrahydroquinolinyl and tetrahydrobenzofuranyl. The heteroaryl and cycloalkyl rings may each be sustitued by one or more substituent groups. The heteroaryl-fused-cycloalkyl group may be attached to the remainder of the compound of formula [1] by any available carbon or nitrogen atom.
"Heteroaryl-fused-heterocycloalkyl" means a monocyclic heteroaryl group, such as pyridyl or furanyl, fused to a heterocycloalkyl group, in which heteroaryl and heterocycloalkyl are as previously described. Exemplary heteroaryl-fused-heterocycloalkyl groups include dihydrodioxinopyridinyl, dihydropyrrolopyridinyl, dihydrofuranopyridinyl and dioxolopyridinyl. The heteroaryl and heterocycloalkyl rings may each be sustitued by one or more substituents groups. The heteroaryl-fused-heterocycloalkyl group may be attached to the remainder of the compound of formula [1] by any available carbon or nitrogen atom.
"Heterocycloalkyl" means: (i) an optionally substituted cycloalkyl group of from 4 to 8 ring members which contains one or more heteroatoms selected from O, S or NR; (ii) a cycloalkyl group of from 4 to 8 ring members which contains CONR and CONRCO (examples of such groups include succinimidyl and 2-oxopyrrolidinyl). The heterocycloalkyl group may be substituted by one or more substituent groups. The heterocycloalkyl group may be attached to the remainder of the compound of formula [1] by any available carbon or nitrogen atom.
"Heterocycloalkylalkyl" means a heterocycloalkyl-alkyl- group in which the heterocycloalkyl and alkyl moieties are as previously described.
"Lower alkyl" as a group means unless otherwise specified, an aliphatic hydrocarbon group which may be straight or branched having 1 to 4 carbon atoms in the chain, i.e. methyl, ethyl, propyl (propyl or *iso*-propyl) or butyl (butyl, *iso-*butyl or *tert*-butyl).
"Sulfonyl" means a -SO₂-alkyl group in which alkyl is as described herein. Exemplary sulfonyl groups include methanesulfonyl.
"Sulfonylamino" means a -NR-sulfonyl group in which R and sulfonyl are as described herein. Exemplary sulfonylamino groups include -NHSO₂CH₃.

R means alkyl, aryl, or heteroaryl as described herein.

"Pharmaceutically acceptable salt" means a physiologically or toxicologically tolerable salt and include, when appropriate, pharmaceutically acceptable base addition salts and pharmaceutically acceptable acid addition salts. For example (i) where a compound of the invention contains one or more acidic groups, for example carboxy groups, pharmaceutically acceptable base addition salts that may be formed include sodium, potassium, calcium, magnesium and ammonium salts, or salts with organic amines, such as, diethylamine, *N*-methyl-glucamine, diethanolamine or amino acids (e.g. lysine) and the like; (ii) where a compound of the invention contains a basic group, such as an amino group, pharmaceutically acceptable acid addition salts that may be formed include hydrochlorides, hydrobromides, phosphates, acetates, citrates, lactates, tartrates, malonates, methanesulphonates and the like.

It will be understood that, as used in herein, references to the compounds of formula [1] are meant to also include the pharmaceutically acceptable salts.

"Prodrug" means a compound which is convertible *in vivo* by metabolic means (e.g. by hydrolysis, reduction or oxidation) to a compound of formula [1]. For example an ester prodrug of a compound of formula [1] containing a hydroxy group may be convertible by hydrolysis *in vivo* to the parent molecule. Suitable esters of compounds of formula [1] containing a hydroxy group, are for example acetates, citrates, lactates, tartrates, malonates, oxalates, salicylates, propionates, succinates, fumarates, maleates, methylene-bis-β-hydroxynaphthoates, gentisates, isethionates, di-*p*-toluoyltartrates, methanesulphonates, ethanesulphonates, benzenesulphonates, p-toluenesulphonates, cyclohexylsulphamates and quinates. As another example an ester prodrug of a compound of formula [1] containing a carboxy group may be convertible by hydrolysis *in vivo* to the parent molecule. Examples of ester prodrugs are those described by F. J. Leinweber, Drug Metab. Res., 1987, 18, 379.

It will be understood that, as used in herein, references to the compounds of formula [1] are meant to also include the prodrug forms.

"Saturated" pertains to compounds and/or groups which do not have any carbon-carbon double bonds or carbon-carbon triple bonds.

The cyclic groups referred to above, namely, aryl, heteroaryl, cycloalkyl, aryl-fused-cycloalkyl, heteroaryl-fused-cycloalkyl, heterocycloalkyl, aryl-fused-heterocycloalkyl, heteroaryl-fused-heterocycloalkyl and cyclic amine may be substituted by one or more substituent groups. Suitable optional substituent groups include acyl (e.g. -COCH₃), alkoxy (e,g, -OCH₃), alkoxycarbonyl (e.g. -COOCH₃), alkylamino (e.g. -NHCH₃), alkylsulfinyl (e.g. -SOCH₃), alkylsulfonyl (e.g. -SO₂CH₃), alkylthio (e.g. -SCH₃), -NH₂, aminoalkyl (e.g. -CH₂NH₂), arylalkyl (e.g. -CH₂Ph or -CH₂-CH₂-Ph), cyano, dialkylamino (e.g. -N(CH₃)₂), halo, haloalkoxy (e.g. -OCF₃ or -OCHF₂), haloalkyl (e.g. -CF₃), alkyl (e.g. -CH₃ or -CH₂CH₃), -OH, -CHO, -NO₂, aryl (optionally substituted with alkoxy, haloalkoxy, halogen, alkyl or haloalkyl), heteroaryl (optionally substituted with alkoxy, haloalkoxy, halogen, alkyl or haloalkyl), heterocycloalkyl, aminoacyl (e.g. - CONH₂, -CONHCH₃), aminosulfonyl (e.g. -SO₂NH₂, -SO₂NHCH₃), acylamino (e.g. - NHCOCH₃), sulfonylamino (e.g. -NHSO₂CH₃), heteroarylalkyl, cyclic amine (e.g. morpholine), aryloxy, heteroaryloxy, arylalkyloxy (e.g. benzyloxy) and heteroarylalkyloxy.

Alkyl, alkylene or alkenylene groups may be optionally substituted. Suitable optional substituent groups include alkoxy (e,g, -OCH₃), alkylamino (e.g. -NHCH₃), alkylsulfinyl (e.g. -SOCH₃), alkylsulfonyl (e.g. -SO₂CH₃), alkylthio (e.g. -SCH₃), -NH₂, aminoalkyl (e.g. -CH₂NH₂), arylalkyl (e.g. -CH₂Ph or -CH₂-CH₂-Ph), cyano, dialkylamino (e.g. -N(CH₃)₂), halo, haloalkoxy (e.g. -OCF₃ or -OCHF₂), haloalkyl (e.g. -CF₃), alkyl (e.g. -CH₃ or -CH₂CH₃), -OH, -CHO, and -NO₂.

Compounds of the invention may exist in one or more geometrical, optical, enantiomeric, diastereomeric and tautomeric forms, including but not limited to *cis-* and *trans-forms, E-*and Z-forms, *R*-, *S*- and *meso*-forms, keto-, and enol-forms. Unless otherwise stated a reference to a particular compound includes all such isomeric forms, including racemic and other mixtures thereof. Where appropriate such isomers can be separated from their mixtures by the application or adaptation of known methods (e.g. chromatographic techniques and recrystallisation techniques). Where appropriate such isomers may be prepared by the application of adaptation of known methods (e.g. asymmetric synthesis).

With reference to formula [1] above, particular and preferred embodiments are described below.

In one preferred embodiment A is selected from a group of formula [2], [3], or [4]:

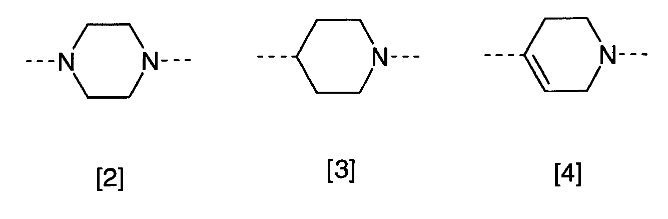

In a preferred embodiment A is a group of formula [2].

In a preferred embodiment B is a phenyl ring.

In a preferred embodiment B is substituted by one or two groups of formula R⁵.

In a further embodiment B is substituted by one or two groups chosen independently from: Cl, Br, methyl, OH, CF₃, or NH₂.

In a preferred embodiment B is substituted by a chloro group.

In a preferred embodiment B is a phenyl ring substituted by at least one substituent selected from fluoro, chloro, bromo, methyl, OH, NH₂, or CF₃.

In a preferred embodiment X represents O.

In a preferred embodiment R¹ is methyl.

In another preferred embodiment R¹ is H.

In a preferred embodiment R² is H.

In another preferred embodiment R² is methyl.

Preferably R² is an optionally substituted C₃₋₆-cycloalkyl; or C₁₋₄ alkyl, substituted with at least one halogen, preferably fluoro.

More preferred R² is trifluoromethyl or cyclopropyl.

In one embodiment R³ and R⁴ are H.

In one embodiment compounds of the invention are:
• 8-chloro-2-methyl-4-(4-methylpiperazin-1-yl)benzo[4,5]furo[3,2-d]pyrimidine
• 8-chloro-4-(4-methylpiperazin-1-yl)benzo[4,5]furo[3,2-d]pyrimidine
• 4-(4-methylpiperazin-1-yl)benzo[4,5]furo[3,2-d]pyrimidine
• 6-chloro-1-(4-methylpiperazin-1-yl)-9H-2,4,9-triazafluorene

In a second embodiment compounds of the invention are:
• 4-(Piperazin-1-yl)benzo[4,5]furo[3,2-d]pyrimidine
• 8-Chloro-2-cyclopropyl-4-(4-methylpiperazin-1-yl)benzo[4,5]furo[3,2-d]pyrimidine
• 8-Chloro-4-(4-methylpiperazin-1-yl)-2-trifluoromethylbenzo[4,5]furo[3,2-d]pyrimidine
• 8-Chloro-4-(1-methylpiperidin-4-yl)benzo[4,5]furo[3,2-d]pyrimidine

### Utilities of the Invention

The present invention provides compounds that antagonise the effects of histamine at the H4 receptor according to the tests described in the Biological Methods section of this document. The present invention also provides compounds that antagonise the effects of histamine at the H4 and H1 receptors. The therapeutic application of these compounds is pertinent to any disease that is known to be at least partially mediated by the activation of the H4 receptor and, optionally, the H1 receptor. For example, these compounds could be beneficial for the treatment of inflammatory diseases, asthma, psoriasis, rheumatoid arthritis, Crohn's disease, inflammatory bowel disease, ulcerative colitis, allergic rhinitis and other allergic diseases, and atopic dermatitis and other dermatological disorders.

The present invention is also concerned with treatment of these conditions, and the use of compounds of the present invention for manufacture of a medicament useful in treating these conditions.

### Combinations

Other compounds may be combined with compounds of this invention of formula [1] for the prevention and treatment of histamine-mediated diseases. Thus the present invention is also concerned with pharmaceutical compositions for preventing and treating histamine-mediated diseases comprising a therapeutically effective amount of a compound of the invention of formula [1] and one or more other therapeutic agents. Suitable therapeutic agents for a combination therapy with compounds of formula [1] include: (1) a corticosteroid, for example fluticasone or budesonide; (2) a β2-adrenoreceptor agonist, for example salmeterol or formeterol; (3) a leukotriene modulator, for example montelukast or pranlukast; (4) anticholinergic agents, for example selective muscarinic-3 (M3) receptor antagonists such as tiotropium bromide; (5) phosphodiesterase-IV (PDE-IV) inhibitors, for example roflumilast or cilomilast; (6) an antitussive agent, such as codeine or dextramorphan; (7) a non-steroidal anti-inflammatory agent (NSAID), for example ibuprofen or ketoprofen and (8) an H1 antagonist or inverse agonist, for example loratidine or cetirizine.

The weight ratio of the compound of the formula (1) to the second active ingredient may be varied and will depend upon the effective dose of each ingredient. Generally, an effective dose of each will be used.

### Pharmaceutical formulations

The present invention is also concerned with pharmaceutical formulations comprising one of the compounds as an active ingredient.

The magnitude of prophylactic or therapeutic dose of a compound of formula [1] will, of course, vary with the nature of the severity of the condition to be treated and with the particular compound of formula [1] and its route of administration. It will also vary according to the age, weight and response of the individual patient. In general, the daily dose range will lie within the range of from about 0.001 mg to about 100 mg per kg body weight of a mammal, preferably 0.01 mg to about 50 mg per kg, and most preferably 0.1 to 10 mg per kg, in single or divided doses. On the other hand, it may be necessary to use dosages outside these limits in some cases.

For use where a composition for the intravenous administration is employed, a suitable dosage range is from about 0.001 mg to about 25 mg (preferably from 0.01 mg to about 1 mg) of a compound of formula [1] per kg of body weight per day.

In the cases where an oral composition is employed, a suitable dosage range is, for example, from about 0.01mg to about 100mg of a compound of formula [1] per day, preferably from about 0.1mg to about 10mg per day. For oral administration, the compositions are preferably provided in the form of tablets containing from 0.01 to 1,000mg, preferably 0.01, 0.05, 0.1, 0.5, 1.0, 2.5, 5.0, 10.0, 15.0, 20.0, 25.0, 30.0, 40.0, 50.0 or 1000.0 milligrams of the active ingredient for the symptomatic adjustment of the dosage to the patient to be treated.

Another aspect of the present invention provides pharmaceutical compositions which comprise a compound of formula [1] and a pharmaceutically acceptable carrier. The term "composition", as in pharmaceutical composition, is intended to encompass a product comprising the active ingredient(s), and the inert ingredient(s) (pharmaceutically acceptable excipients) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of formula [1], additional active ingredient(s), and pharmaceutically acceptable excipients.

Any suitable route of administration may be employed for providing a mammal, especially a human, with an effective dosage of a compound of the present invention. For example, oral, rectal, topical, parenteral, ocular, pulmonary, nasal, and the like may be employed. Dosage forms include tablets, troches, dispersions, suspensions, solutions, capsules, creams, ointments, aerosols, and the like.

The pharmaceutical compositions of the present invention comprise a compound of formula [1] as an active ingredient or a pharmaceutically acceptable salt thereof, and may also contain a pharmaceutically acceptable carrier and optionally other therapeutic ingredients. The term "pharmaceutically acceptable salts" refers to salts prepared from pharmaceutically acceptable non-toxic bases or acids including inorganic bases or acids and organic bases or acids.

The compositions include compositions suitable for oral, rectal, topical, parenteral (including subcutaneous, intramuscular, and intravenous), ocular (ophthalmic), pulmonary (aerosol inhalation), or nasal administration, although the most suitable route in any case will depend on the nature and severity of the conditions being treated and on the nature of the active ingredient. They may be conveniently presented in unit dosage form and prepared by any of the methods well-known in the art of pharmacy.

For administration by inhalation, the compounds of the present invention are conveniently delivered in the form of an aerosol spray presentation from pressurized packs or nebulizers. The compounds may also be delivered as powders which may be formulated and the power composition may be inhaled with the aid of insufflation powder inhaler device. The preferred delivery systems for inhalation are metered dose inhalation (MDI) aerosol, which may be formulated as a suspension or solution of a compound of formula [1] in suitable propellants, such as fluorocarbons or hydrocarbons and dry powder inhalation (DPI) aerosol, which can be formulated as a dry powder of a compound of formula [1] with or without additional excipients.

Suitable topical formulations of a compound of formula [1] include transdermal devices, aerosols, creams, ointments, lotions, dusting powders and the like.

In practical use, the compounds of formula [1] can be combined as the active ingredient in intimate admixture with a pharmaceutical carrier according to conventional pharmaceutical compounding techniques. The carrier may take a wide variety of forms depending on the form of preparation desired for administration, e.g. oral or parenteral (including intravenous). In preparing the compositions for oral dosage form, any of the usual pharmaceutical media may be employed, such, as, for example, water, glycols, oils, alcohols, flavouring agents, preservatives, colouring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, capsules and tablets, with the solid oral preparations being preferred over the liquid preparations. Because of their ease of administration, tablets and capsules represent the most advantageous oral dosage unit form in which case solid pharmaceutical carriers are obviously employed. If desired, tablets may be coated by standard aqueous or nonaqueous techniques.

In addition to the common dosage forms set out above, the compounds of formula [1] may also be administered by controlled release means and/or delivery devices such as those described in U.S patents 3845770, 3916899, 3536809, 3598123, 3630200 and 4008719.

Pharmaceutical compositions of the present invention suitable for oral administration may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or moulding, optionally with one or more accessory ingredients. Compressed tablets may be prepared by compressing in a suitable machine, the active ingredient in a free-flowing form such as powder or granules, optionally mixed with a binder, lubricant, inert diluent, surface active or dispersing agent. Moulded tablets may be made by moulding in a suitable machine, a mixture of the powdered compound moistened with an inert liquid diluent. Desirably, each tablet contains from about 1 mg to about 500 mg of the active ingredient and each cachet or capsule contains from about 1 to about 500 mg of the active ingredient.

The following are examples of representative pharmaceutical dosage forms for the compounds of formula [1]:
Injectable Suspension (I.M.):

| | |
|---|---|
| Compound of formula [1] | 10 mg / mL |
| Methylcellulose | 5,0 mg / mL |
| Tween 80 | 0,5 mg / mL |
| Benzyl alcohol | 9,0 mg / mL |
| Benzalkonium chloride | 1,0 mg / mL |

Plus water for injection to a total volume of 1mL
500 mg Tablet:

| | |
|---|---|
| Compound of formula [1] | 25 mg / tablet |
| Microcrystalline Cellulose | 415 mg/mL |
| Povidone | 14,0 mg/mL |
| Pregelatinized Starch | 43,5 mg/mL |
| Magnesium Stearate | 2,5 mg/mL |

600 mg Capsule:

| | |
|---|---|
| Compound of formula [1] | 25 mg / tablet |
| Lactose Powder | 573,5 mg / tablet |
| Magnesium Stearate | 1,5 mg / tablet |

Aerosol:

| | |
|---|---|
| Compound of formula [1] | 24 mg / canister |
| Lecithin, NF Liq. Conc. | 1,2 mg / canister |
| Trichlorofluoromethane, NF | 4,025 g / canister |
| Dichlorodifluoromethane, NF | 12,15 g / canister |

Compounds of formula [1] may be used in combination with other drugs that are used in the treatment/prevention/suppression or amelioration of the diseases or conditions for which compounds of formula [1] are useful. Such other drugs may be administered, by a route and in an amount commonly used therefore, contemporaneously or sequentially with a compound of formula [1]. When a compound of formula [1] is used contemporaneously with one or more other drugs, a pharmaceutical composition containing such other drugs in addition to the compound of formula [1] is preferred. Accordingly, the pharmaceutical compositions of the present invention include those that also contain one or more other active ingredients, in addition to a compound of formula [1].

### Methods of Synthesis

The present invention is also concerned with processes for preparing the compounds of this invention.

The compounds of formula [1] of the present invention can be prepared according to the procedures of the following schemes and examples, using appropriate materials, and are further exemplified by the following specific examples. Moreover, by utilising the procedures described with the disclosure contained herein, one of ordinary skill in the art can readily prepare additional compounds of the present invention claimed herein. The compounds illustrated in the examples are not, however, to be construed as forming the only genus that is considered as the invention. The examples further illustrate details for the preparation of the compounds of the present invention. Those skilled in the art will readily understand that known variations of the conditions and processes of the following preparative procedures can be used to prepare these compounds.

The compounds of the invention of formula [1] may be isolated in the form of their pharmaceutically acceptable salts, such as those described previously herein above. The free acid form corresponding to isolated salts can be generated by neutralisation with a suitable acid such as acetic acid and hydrochloric acid and extraction of the liberated free acid into an organic solvent followed by evaporation. The free acid form isolated in this manner can be further converted into another pharmaceutically acceptable salt by dissolution in an organic solvent followed by addition of the appropriate base and subsequent evaporation, precipitation, or crystallisation.

It may be necessary to protect reactive functional groups (e.g. hydroxy, amino, thio or carboxy) in intermediates used in the preparation of compounds of formula [1] to avoid their unwanted participation in a reaction leading to the formation of compounds of formula [1]. Conventional protecting groups, for example those described by T. W. Greene and P. G. M. Wuts in "Protective groups in organic chemistry" John Wiley and Sons, 1999, may be used.

Compounds of the invention of formula [1a], in which group A is attached to the pyrimidine ring through a nitrogen atom, may conveniently be prepared by the reaction in an inert solvent, usually under elevated temperatures, of a cyclic amine of formula [5] and a compound of formula [6] in which R⁸ represents a suitable leaving group; suitable leaving groups at R⁸ include chloro, bromo, alkylsulphinyl, and alkylsulphonyl.

Alternatively the reaction of intermediate [6], in which R⁸ is a halo group such as chloro or bromo, with an cyclic amine intermediate of formula [5] may be achieved in the presence of a palladium catalyst such as a mixture of palladium bis(trifluoroacetate) and tri(*tert-*butyl)phosphine.

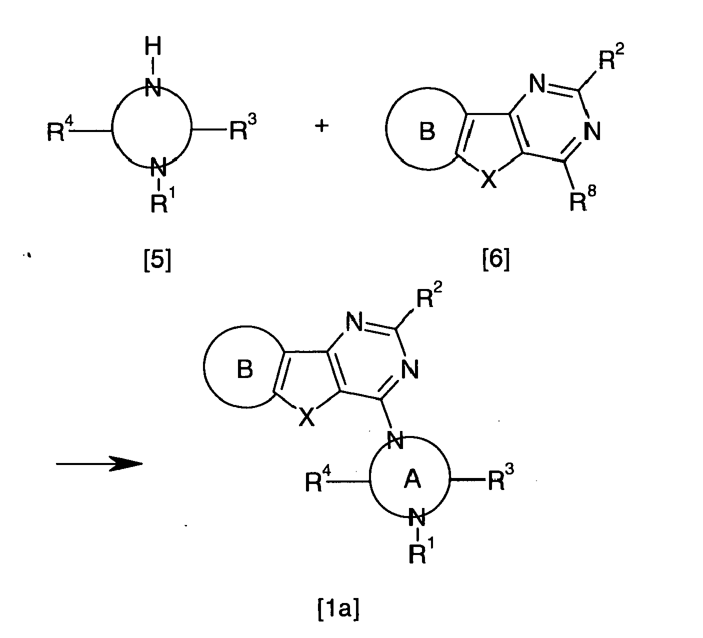

It will be understood by those practiced in the art that the transformation of intermediate [6] to compound [1a] by reaction with cyclic amine [5] when R¹ is H may require the presence of a suitable protecting group, for example benzyl, benzoyl or *tert-*butyloxycarbonyl, as may prove most convenient. It is to be understood that if the reaction is carried out on a protected form of cyclic amine [5] an appropriate deprotection step will be required to obtain the desired compound [1a] of the invention in which R¹ is H.

Compounds of the invention of formula [1b] in which group A is attached to the pyrimidine ring through an unsaturated carbon atom (for example A is a group of formula [4]) may be conveniently prepared by the reaction between an intermediate of formula [6] in which R⁸ is a halo atom such as chloro or bromo, and a substituted alkene of formula [7], in which R⁹ is a suitable metal-containing group such as a boronate ester or a trialkyl- or triarylstanne, in the presence of a suitable palladium catalyst such as tris(dibenzylideneacetone)dipalladium.

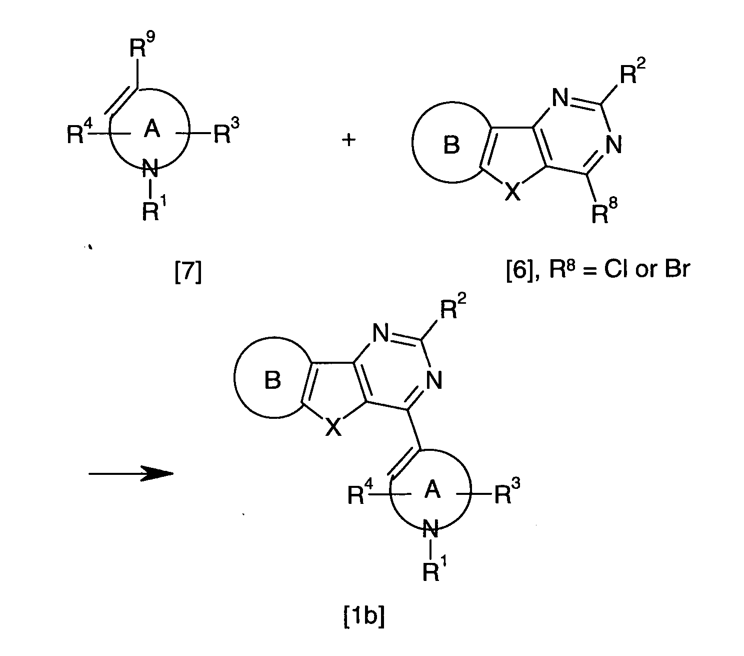

It will be understood by those practiced in the art that the transformation of intermediate [6] to compound [1b] by reaction with cyclic amine [7] when R¹ is H may require the presence of a suitable protecting group, for example benzyl, benzoyl or *tert-*butyloxycarbonyl, as may prove most convenient. It is to be understood that if the reaction is carried out on a protected form of cyclic amine [7] an appropriate deprotection step will be required to obtain the desired compound [1b] of the invention in which R¹ is H.

Compounds of the invention of formula [1c] in which group A is attached to the pyrimidine ring through a saturated carbon atom (for example A is a group of formula [3]) may be conveniently prepared by the reduction of compounds of formula [1b], by, for example, catalytic hydrogenation.

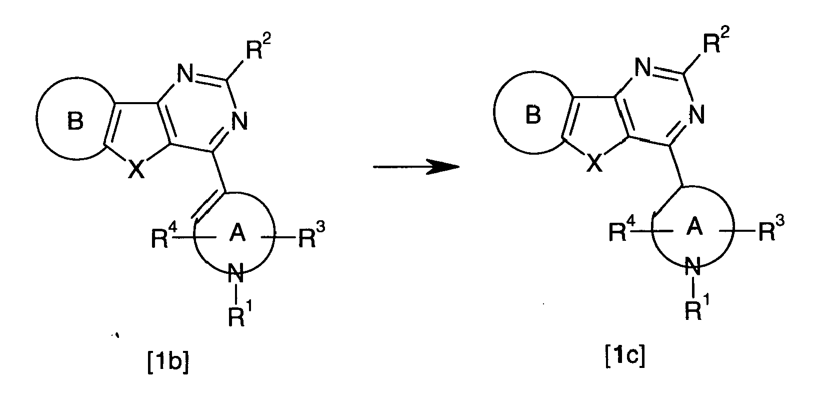

Intermediate compounds of formula [6] where R⁸ = chloro or bromo may be prepared, for example, by the reaction of a compound of formula [8] with a suitable halogenating agent, for example phosphorus oxychloride or phosphorus oxybromide.

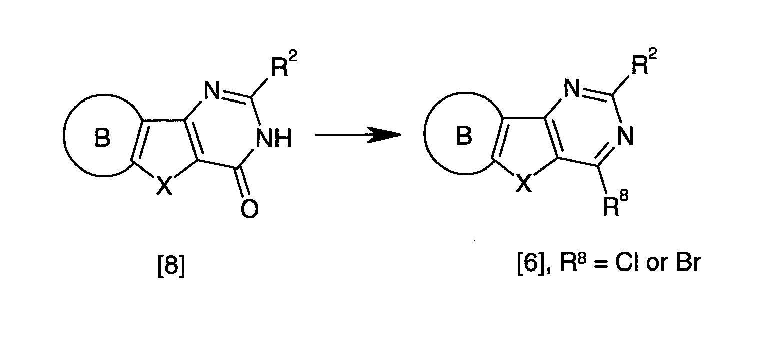

Intermediate compounds of formula [8] may be prepared, for example, from compounds of formula [9], where R⁹ = C(=O)NH₂, CN, or C(=O)Oalkyl, by reaction with a suitable condensing agent. Where R² is H suitable condensing agents include formic acid, formamide, and trialkyl orthoformates; where R² is alkyl suitable condensing agents include symmetrical alkyl anhydrides and alkyl amides; and where R² is aryl or heteroaryl suitable condensing agents include aryl chlorides and arylaldehydes.

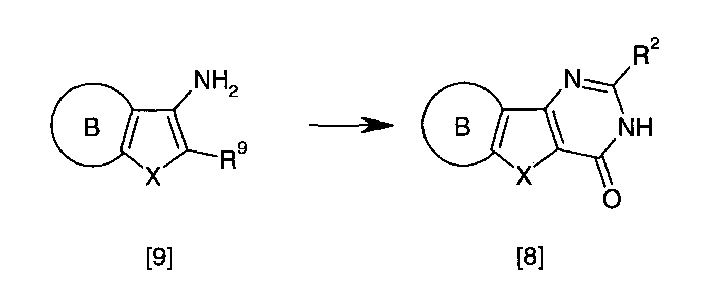

### Biological methods

Compounds of the invention of formula [1] can be tested using the following biological test methods to determine their ability to displace histamine from the H4 receptor and for their ability to antagonise the functional effects of histamine at the H4 receptor in a whole cell system.

Radioligand binding assay using histamine H4 receptor transfected CHO K1 membranes.

The receptor binding assay is performed in a final volume of 150 µL binding buffer (50 mM Tris (pH 7.4), 5 mM MgCl2) using 18nM [2,5-³H]-histamine dihydrochloride (Amersham Biosciences UK Ltd) as the radioligand. Ligands are added in assay buffer containing a constant volume of DMSO (1% v/v). Total binding is determined using 1% v/v of DMSO in assay buffer and non-specific binding is determined using 100 µM of unlabeled histamine dihydrochloride (Sigma). The reaction is initiated with 20 µg histamine H4 receptor membranes (Euroscreen, Belgium) and the mixture incubated for 90 minutes at 25°C. The reaction is terminated by rapid filtration through GF/B filters pre-blocked with PEI (1 % v/v) using a Packard Cell harvester and the filter washed with 2 x 500 µL / well of cold wash buffer (50 mM Tris (pH 7.4), 5 mM MgCl2, 0.5 M NaCl). The residual radioligand bound to the filter was determined using a Topcount liquid scintillation counter (Perkin Elmer). Compound IC₅₀ values were determined using an 8-point dose response curve in duplicate with a semi-log compound dilution series. IC₅₀ calculations were performed using Excel and XL fit (Microsoft) and this value is used to determine a Kᵢ value for the test compound using the Cheng-Prusoff equation. Compounds of the invention typically demonstrate Ki values in this assay of ≤ 10 µM.

Functional assay using histamine H4 receptor transfected CHO K1 membranes.

The GTPγS binding assay is used as a measure of the functional activation of the histamine H4 receptor using membranes prepared from CHO K1 cells stably transfected with the cDNA for the histamine H4 receptor (Euroscreen, Belgium). The assay was performed in a 96 well Isoplate (Perkin Elmer) in a final volume of 200 µL assay buffer (20 mM HEPES (pH 7.4), 100 mM NaCl, 10 mM MgCl2, 10 µg/ml saponin and 10 µM GDP) using 0.1nM GTPγ[³⁵S] (Amersham Biosciences UK Ltd) to measure functional incorporation, and in the case of antagonist studies 150 nM histamine dihydrochloride (EC₈₀ for histamine dihydrochloride) to determine maximal incorporation of GTPγ[³⁵S]. Compounds were added in assay buffer containing a constant volume of DMSO (1 % v/v). Total incorporation was determined in the presence of 1 % v/v of DMSO in assay buffer and non-specific binding is determined using 10 µM of unlabeled GTPγS (Sigma). The incorporation was initiated with 15 µg histamine H4 receptor membranes (Euroscreen, Belgium) and the mixture incubated for 5 minutes at 30 °C. Wheat-Germ agglutinin-coated SPA beads (0.75 mg, Amersham Biosciences UK Ltd) were added and the mixture and incubated for 30 minutes at 30 °C. The plate was centrifuged at 1000 x g for 10 minutes at 30 °C and radioactive incorporation counted in a MicroBeta Counter (Wallac).

### Examples

The invention will now be described in detail with reference to the following examples. It will be appreciated that the invention is described by way of example only and modification of detail may be made without departing from the scope of the invention.

¹H NMR spectra were recorded at ambient temperature using either a Varian Unity Inova (400MHz) spectrometer or a Bruker Advance DRX (400MHz) spectrometer, both with a triple resonance 5mm probe. Chemical shifts are expressed in ppm relative to tetramethylsilane. The following abbreviations have been used: br = broad signal, s = singlet, d = doublet, dd = double doublet, t = triplet, q = quartet, m = multiplet.

High Pressure Liquid Chromatography - Mass Spectrometry (LCMS) experiments to determine retention times and associated mass ions were performed using the following methods:
Method A: Experiments performed on a Micromass Platform LCT spectrometer with positive ion electrospray and single wavelength UV 254nm detection using a Higgins Clipeus C18 5µm 100 x 3.0mm column and a 1 mL / minute flow rate. The initial solvent system was 95% water containing 0.1% formic acid (solvent A) and 5% acetonitrile containing 0.1% formic acid (solvent B) for the first minute followed by a gradient up to 5% solvent A and 95% solvent B over the next 14 minutes. The final solvent system was held constant for a further 5 minutes.
Method B: Experiments performed on a Micromass Platform LC spectrometer with positive and negative ion electrospray and ELS/Diode array detection using a Phenomenex Luna C18(2) 30 x 4.6mm column and a 2 ml / minute flow rate. The solvent system was 95% solvent A and 5% solvent B for the first 0.50 minutes followed by a gradient up to 5% solvent A and 95% solvent B over the next 4 minutes. The final solvent system was held constant for a further 1 minute.

### Microwave experiments were carried out using a Personal Chemistry Smith Synthesizer™, which uses a single-mode resonator and dynamic field tuning, both of which give reproducibility and control. Temperatures from 40-250°C can be achieved, and pressures of up to 20bar can be reached.

**Intermediate 1:** 5-Chloro-2-cyanomethoxybenzonitrile 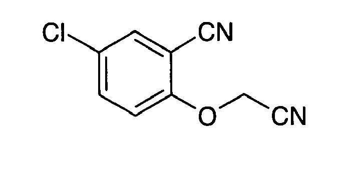 A solution of 4-chloro-2-cyanophenol (2.5g) in acetone was treated with potassium carbonate (2.3g), followed by bromoacetonitrile (1.2mL) and the resulting mixture was stirred at room temperature overnight. The mixture was filtered and the filtrate was evaporated to dryness to give 5-chloro-2-cyanomethoxybenzonitrile (3.3g) as a pale yellow solid.

¹H NMR (DMSO-d₆): δ 5.40 (s, 2H), 7.45 (d, 1H), 7.85 (dd, 1H), 8.05 (d, 1H).

### Intermediate 2: Ethyl (4-chloro-2-cyanophenoxy)acetate

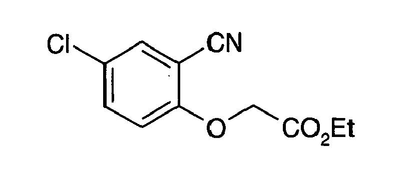

A solution of 4-chloro-2-cyanophenol (5.0g) in N,N-dimethylformamide (20mL) was added dropwise to an ice-cooled suspension of sodium hydride (60% oil dispersion, 1.4g) in N,N-dimethylformamide (165mL). The resultant mixture was stirred at 0-5°C for 40 minutes, then ethyl bromoacetate (3.9mL) was added and stirring was continued for 1.5 hours. Ethyl acetate and hydrochloric acid (1M) were added and the layers were separated. The organic layer was washed with water, aqueous sodium bicarbonate solution and brine, then dried over sodium sulphate and filtered. The filtrate was evaporated to give crude ethyl (4-chloro-2-cyanophenoxy)acetate (7.8g) as a yellow oil which was used without further purification.

### Intermediate 3: 3-Amino-5-chlorobenzofuran-2-carbonitrile

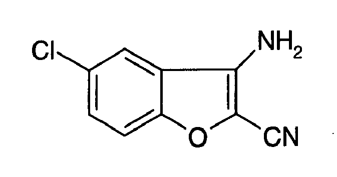

A solution of 5-chloro-2-cyanomethoxybenzonitrile (intermediate 1, 3.3g) in N,N-dimethylformamide (50mL) was treated with potassium carbonate (2.2g) and the mixture was stirred at 100°C for about 8 hours. After cooling to room temperature the reaction mixture was poured onto water and the resulting precipitate was collected by filtration, washed with water, and dried to give 3-amino-5-chlorobenzofuran-2-carbonitrile (3.0g) as a yellow solid.
¹H NMR (DMSO-d₆): δ 6.7 (br s, 2H), 7.55 (m, 2H), 8.05 (m, 1H).

### Intermediate 4: 3-Amino-5-chlorobenzofuran-2-carboxamide

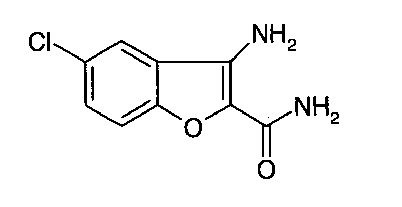

A mixture of 3-amino-5-chlorobenzofuran-2-carbonitrile (intermediate 3, 0.192g) in concentrated sulphuric acid (2mL) was stirred at room temperature for 2 hours. The mixture was poured into a mixture of ice and water and stirred for 10 minutes. The resultant solid was collected by filtration, suspended in toluene and evaporated to dryness to give 3-amino-5-chlorobenzofuran-2-carboxamide (0.15g) as an orange powder.
LCMS (method B): retention time 2.48 minutes, (M+H⁺) 211.

### Intermediate 5: Ethyl 3-amino-5-chlorobenzofuran-2-carboxylate

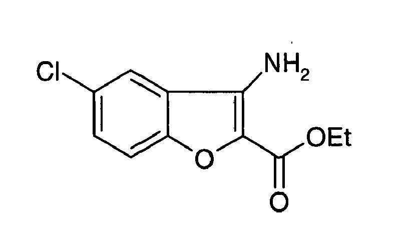

A solution of ethyl (4-chloro-2-cyanophenoxy)acetate (intermediate 2, 0.9g) in dry tetrahydrofuran (5mL) was added dropwise to a suspension of potassium *tert*-butoxide (0.422g) in dry tetrahydrofuran (5mL) under an atmosphere of argon. The resultant thick mixture was stirred at room temperature for 1.5 hours. Water was added followed by acetic acid and the resultant suspension was extracted with chloroform, washed with water, aqueous sodium bicarbonate and brine, then dried over sodium sulphate and filtered. The filtrate was evaporated to give ethyl 3-amino-5-chlorobenzofuran-2-carboxylate (0.4g) as an off-white solid.
¹H NMR (CDCl₃): δ 1.45 (t, 3H), 4.45 (q, 2H), 4.95 (br s, 2H), 7.45 (s, 2H), 7.55 (s, 1H).

### Intermediate 6: 4,8-Dichloro-2-methylbenzo[4,5]furo[3,2-d]pyrimidine

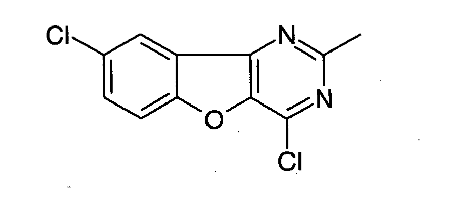

N,N-Dimethylacetamide (0.87mL) was added cautiously, under an atmosphere of nitrogen, to ice-cooled phosphorus oxychloride (2.2mL). When addition was complete the mixture was stirred with ice cooling for a further 30 minutes, then 3-amino-5-chlorobenzofuran-2-carbonitrile (intermediate 3, 1.5g) was added and the resulting mixture stirred at 50°C for 2 hours. After cooling to room temperature the mixture was evaporated to low bulk, diluted with water and neutralised by the careful addition of solid sodium hydrogen carbonate. The mixture was extracted with chloroform (x 3), and the combined extracts were washed with water, dried over magnesium sulphate and filtered. The filtrate was evaporated to give a brown solid (1.3g). Purification of this mixture by flash chromatography eluting with a mixture of ethyl acetate and pentane (1:10) gave 4,8-dichloro-2-methylbenzo[4,5]furo[3,2-d]pyrimidine (0.16g) as a white solid.
¹H NMR (DMSO-d₆): δ 2.75 (s, 3H), 7.90 (dd, 1H), 8.00 (dd, 1H), 8.30 (dd, 1H).

### Intermediate 7: 8-Chloro-3H-benzo[4,5]furo[3,2-d]pyrimidin-4-one

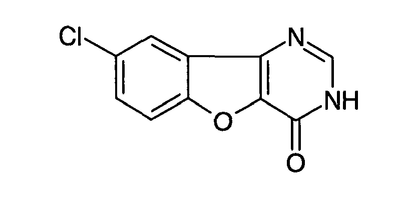

A suspension of ethyl 3-amino-5-chlorobenzofuran-2-carboxylate (intermediate 5, 0.3g) in triethyl orthoformate (2mL) was irradiated in a microwave at 200°C for 10 minutes. The resultant yellow solution was evaporated to dryness and the residue was dissolved in a solution of ammonia in methanol (2M, 2mL). The mixture was carefully irradiated in a microwave at 140°C for 10 minutes and the resultant precipitate was collected by filtration and washed with diethyl ether. The filtrate was evaporated to dryness, the residue was triturated with acetonitrile and the solid was collected by filtration. The solids were combined to give 8-chloro-3H-benzo[4,5]furo[3,2-d]pyrimidin-4-one (0.175 g) as a grey powder.
¹H NMR (DMSO-D₆): δ 7.6 (d, 1H), 7.9 (d, 1H), 8.1 (s, 1H), 8.25 (s, 1H).

### Intermediate 8: 8-Chloro-2-cyclopropyl-3H-benzo[4,5]furo[3,2-d]pyrimidin-4-one

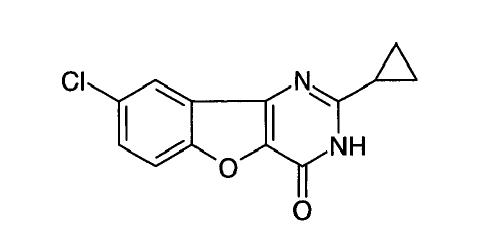

Hydrogen chloride gas was bubbled through a solution of ethyl 3-amino-5-chlorobenzofuran-2-carboxylate (intermediate 5, 0.5g) in cyclopropanecarbonitrile (15mL) for 1.5 hours. The resultant mixture was concentrated *in vacuo* and the residue was dissolved in toluene and evaporated three times. The residue was dissolved in ethanol (11mL) and an aqueous solution of sodium hydroxide (6%, 3.65mL) was added. The mixture was stirred and heated at reflux for 1 hour then cooled to room temperature and the resultant solid was collected by filtration and washed with ethanol to give 8-chloro-2-cyclopropyl-3H-benzo[4,5]furo[3,2-d]pyrimidin-4-one (0.133g) as a tan-coloured solid.
¹H NMR (DMSO-d₆): δ 1.1 (m, 4H), 2.05 (m, 1H), 7.65 (d, 1H), 7.85 (d, 1H), 7.95 (s, 1H).

### Intermediate 9: 8-Chloro-2-trifluoromethyl-3H-benzo[4,5]furo[3,2-d]pyrimidin-4-one

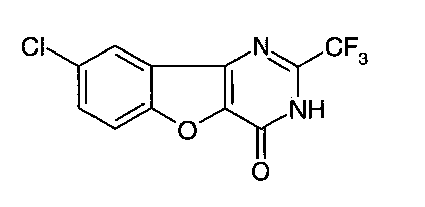

A mixture of 3-amino-5-chlorobenzofuran-2-carboxamide (intermediate 4, 0.051g) and ethyl trifluoroacetate (0.235mL) was added to a solution of sodium ethoxide in ethanol (1M, 2mL) and the mixture was irradiated in a microwave at 140°C for 10 minutes. The resultant mixture was evaporated to dryness and the residue was dissolved in water and treated with concentrated hydrochloric acid. The mixture was evaporated to dryness to give crude 8-chloro-2-trifluoromethyl-3H-benzo[4,5]furo[3,2-d]pyrimidin-4-one as a brown powder, which was used directly without further purification.

### Intermediate 10: 4,8-Dichlorobenzo[4,5]furo[3,2-d]pyrimidine

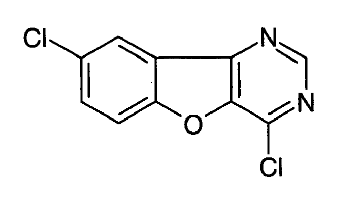

A mixture of 8-chloro-3H-benzo[4,5]furo[3,2-d]pyrimidin-4-one (intermediate 7, 0.175g) and phosphorus oxychloride (2mL) was irradiated in a microwave at 180°C for 15 minutes. The solution was evaporated to dryness and the residue was treated with aqueous ammonia solution. The resultant solid was collected by filtration and triturated with acetonitrile, sonicated with water, collected by filtration and finally triturated with acetonitrile to give 4,8-dichlorobenzo[4,5]furo[3,2-d]pyrimidine (0.107g) as a beige solid.
¹H NMR (CDCl₃): δ 7.7 (d, 1H), 7.75 (d, 1H), 8.25 (s, 1H), 9.05 (s, 1H).

By proceeding in a similar manner the following compounds were prepared from the appropriate starting materials.

### Intermediate 11: 4,8-Dichloro-2-cyclopropylbenzo[4,5]furo[3,2-d]pyrimidine

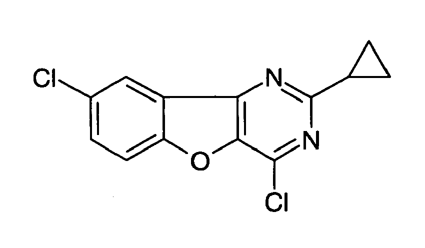

¹H NMR (CDCl₃): δ 1.1 (m, 2H), 1.2 (m, 2H), 2.4 (m, 1H), 7.6 (d, 1H), 7.65 (d, 1H), 8.2 (s, 1H).
starting from 8-chloro-2-cyclopropyl-3H-benzo [4,5]furo[3,2-d]pyrimidin-4-one (intermediate 8)

### Intermediate 12: 4,8-Dichloro-2-trifluoromethylbenzo[4,5]furo[3,2-d]pyrimidine,

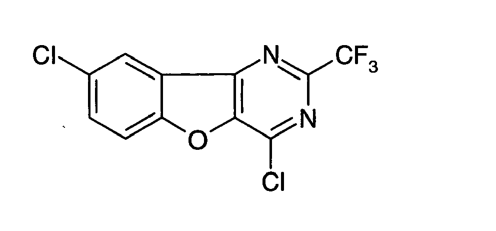

which was used without further purification starting from 8-chloro-2-trifluoromethyl-3H-benzo[4,5]furo[3,2-d]pyrimidin-4-one (intermediate 9)

### Intermediate 13: tert-Butyl 4-(8-chlorobenzo[4,5]furo[3,2-d]pyrimidin-4-yl)-piperidine-1-carboxylate

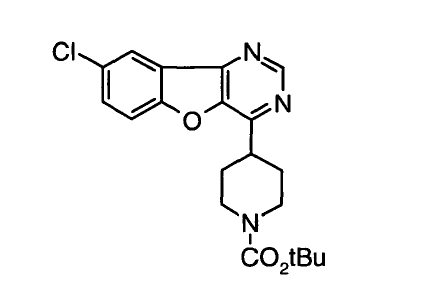

A solution of chlorotrimethylsilane (0.044mL) and 1,2-dibromoethane (0.031mL) in N,N-dimethylacetamide (0.5mL) was added dropwise to a suspension of zinc dust (0.25g) in N,N-dimethylacetamide (0.5mL) under an atmosphere of argon. The mixture was stirred for 30 minutes then treated with a solution of *tert*-butyl 4-iodopiperidine-1-carboxylate (0.962g) in N,N-dimethylacetamide (2mL). This mixture was stirred at room temperature for 45 minutes then filtered directly into a degassed mixture of 4,8-dichlorobenzo[4,5]furo[3,2-d]pyrimidine (intermediate 10, 0.239g), [1,1-bis(diphenylphosphino)ferrocene]dichloropalladium (II) complex with dichloromethane (1:1) (0.049g) and copper (I) iodide (0.023g) in N,N-dimethylacetamide (2mL). The mixture was stirred and heated at 80°C under an atmosphere of argon overnight. After cooling, the mixture was diluted with methanol and passed through an Isolute® SCX-2 column eluting with methanol. The combined fractions were evaporated to dryness and the residue was dissolved in a mixture of methanol and ethyl acetate (1:9), washed with saturated aqueous ammonium chloride solution, water, and brine then dried over sodium sulphate and filtered. The filtrate was evaporated to dryness and the residue was purified by chromatography on silica eluting with a mixture of diethyl ether and n-pentane (1:3, increasing to 1:1) to give *tert*-butyl 4-(8-chlorobenzo[4,5]furo[3,2-d]pyrimnidin-4-yl)piperidine-1-carboxylate (0.11 g) as a yellow powder.
¹H NMR (CDCl₃): δ 1.5 (s, 9H), 2.0 (m, 4H), 2.95 (br, 2H), 3.45 (m, 1H), 4.3 (br, 2H), 7.6 (d, 1H), 7.65 (d, 1H), 8.2 (s, 1H), 9.15 (s, 1H).

### Example 1: 8-Chloro-2-methyl-4-(4-methylpiperazin-1-yl)benzo[4,5]furo[3,2-d]pyrimidine

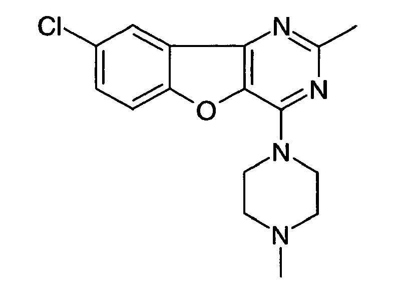

A mixture of 4,8-dichloro-2-methylbenzo[4,5]furo[3,2-d]pyrimidine (intermediate 6) (0.088g) and 1-methylpiperazine (1mL) was heated at 80°C for 30 minutes, then poured onto iced water. The resultant precipitate was collected by filtration, washed with water, and dried to give a white solid (0.032g). The aqueous filtrate was extracted with ethyl acetate (x3), and the combined organic layers were washed with brine, dried over magnesium sulphate and filtered. The filtrate was evaporated to give a pale yellow solid (0.18g). The combined solids were dissolved in methanol and loaded onto an Isolute® SCX-2 column. The column was eluted with methanol to remove unwanted by-products, then with a solution of ammonia in methanol (2M). Evaporation of the eluant gave 8-chloro-2-methyl-4-(4-methylpiperazin-1-yl)benzo[4,5]furo[3,2-d]pyrimidine (0.051g) as a white solid.
¹H NMR (DMSO-d6): δ 2.20 (s, 3H), 2.45 (m, 4H), 2.50 (s, 3H), 3.95 (m, 4H), 7.65 (dd, 1H), 7.75 (dd, 1H), 8.00 (dd, 1H).
LCMS (method A): retention time 6.5 minutes, (M+H⁺) 317.

### Example 2: 8-Chloro-2-cyclopropyl-4-(4-methylpiperazin-1-yl)benzo[4,5]furo[3,2-d]pyrimidine

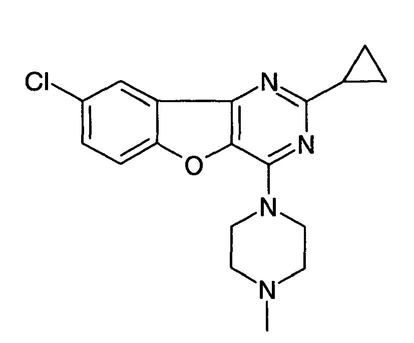

A mixture of 4,8-dichloro-2-cyclopropylbenzo[4,5]furo[3,2-d]pyrimidine (intermediate 11, 0.04g), diethylaminomethyl polystyrene (3.2mmol/g, 0.134g) and 1-methylpiperazine (0.032mL) in ethanol (1mL) was irradiated in a microwave at 120°C for ten cycles of 30 seconds, cooling to 60°C between each cycle. The mixture was diluted with ethanol and the solid was filtered off and washed with ethanol. The filtrate was loaded onto an Isolute® SCX-2 column eluting with methanol to remove the unwanted by-products, then with a solution of ammonia in methanol (2M). The eluant was evaporated to dryness and the residue was purified on an Isolute® NH₂ column eluting with a mixture of diethyl ether and n-pentane (1:1) to give 8-chloro-2-cyclopropyl-4-(4-methylpiperazin-1-yl)benzo[4,5]furo[3,2-d]pyrimidine (0.035g) as a white solid.
¹H NMR (CDCl₃): δ 0.95 (m, 2H), 1.1 (m, 2H), 2.2 (m, 1H), 2.35 (s, 3H), 2.55 (m, 4H), 4.1 (m, 4H), 7.45 (d, 1H), 7.5 (d, 1H) 8.1 (s, 1H).
LCMS (method A): retention time 5.87 minutes, (M+H⁺) 343.

By proceeding in a similar manner the following compound was prepared from the appropriate starting material.

### Example 3: 8-Chloro-4-(4-methylpiperazin-1-yl)-2-trifluoromethylbenzo[4,5]furo-[3,2-d]pyrimidine

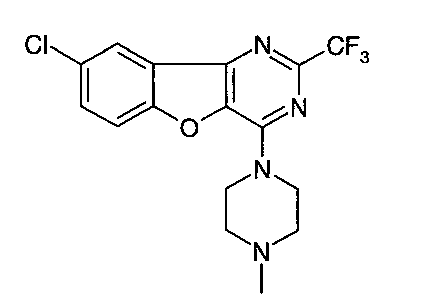

¹H NMR (CDCl₃): δ 2.4 (s, 3H), 2.6 (m, 4H), 4.2 (m, 4H), 7.55 (d, 1H), 7.6 (d, 1H), 8.2 (s, 1H).
LCMS (method A): retention time 7.34 minutes, (M+H⁺) 371.
starting from 4,8-dichloro-2-trif!uoromethylbenzo[4,5]furo[3,2-d]pyrimidine (intermediate 12).

### Example 4: 8-Chloro-4-(1-methylpiperidin-4-yl)benzo[4,5]furo[3,2-d]pyrimidine

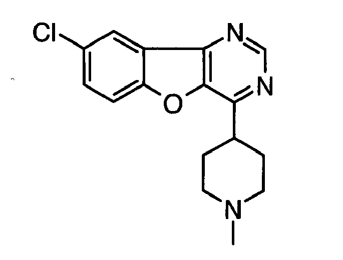

A mixture of *tert*-butyl 4-(8-chlorobenzo[4,5]furo[3,2-d]pyrimidin-4-yl)piperidine-1-carboxylate (intermediate 13, 0.061g), formic acid (1.1mL) and aqueous formaldehyde (37%, 0.09mL) was irradiated in a microwave at 150°C for 5 minutes. The mixture was diluted with methanol and loaded onto an Isolute® SCX-2 column eluting with methanol to remove the unwanted by-products, then with a solution of ammonia in methanol (2M). The eluant was evaporated to give 8-chloro-4-(1-methylpiperidin-4-yl)benzo[4,5]furo[3,2-d]pyrimidine (0.04g) as a pale yellow solid.
¹H NMR (CDCl₃): δ 2.05 (m, 2H), 2.1-2.35 (m, 4H), 2.4 (s, 3H), 3.1 (dd, 2H) 3.2 (m, 1H), 7.6 (d, 1H) 7.65 (d, 1H), 8.2 (s, 1H), 9.15 (s, 1H).

LCMS (method A) retention time 5.72 minutes, (M+H⁺) 302.

## Claims

1. A compound of structural formula [1]: 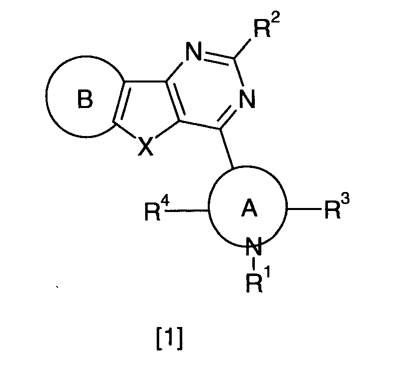 in which:
A represents a fully saturated or partially unsaturated ring of 5 to 7 atoms, at least one of which is a nitrogen atom;
B represents aryl or heteroaryl ring of 5 to 6 atoms, wherein B is optionally substituted with one up to three groups of formula R⁵, where R⁵ represents independently: H, F, Cl, Br, I, C₁₋₄-alkyl, C₃₋₆-cycloalkyl, heterocycloalkyl, C₁₋₄-alkoxy, C₃₋₆-cycloalkoxy, OH, OCF₃, CF₃, cyano, or NR⁶R⁷, R⁶ and R⁷ being independently H or C₁₋₄-alkyl;
X represents O, NH, S, or CH₂;
R¹ represents H, or C₁₋₄-alkyl;
R² represents H, optionally substituted C₁₋₄-alkyl, optionally substituted C₃₋₆-cycloalkyl, or optionally substituted aryl or heteroaryl;
R³ and R⁴ represent independently H, or C₁₋₂-alkyl; or R³ and R⁴ taken together may represent a C₁₋₄-alkylene group;
and corresponding *N*-oxides, pharmaceutically acceptable salts, solvates and prodrugs of such compounds.

2. A compound according to claim 1, wherein R² is an optionally substituted C₃₋₆-cycloalkyl; or C₁₋₄ alkyl, substituted with at least one halogen, preferably fluoro.

3. A compound according to claim 1 or 2, wherein R² is cyclopropyl or CF₃.

4. A compound according to claim 1, selected from the group consisting of:
8-Chloro-2-cyclopropyl-4-(4-methylpiperazin-1-yl)benzo[4,5]furo[3,2-d]pyrimidine; and
8-Chloro-4-(4-methylpiperazin-1-yl)-2-trifluoromethylbenzo[4,5]furo[3,2-d]pyrimidine.

5. A compound according to any of claims 1 to 4 for use as a medicament.

6. A pharmaceutical composition comprising a compound according to claim any of claims 1 to 4 and a pharmaceutically acceptable carrier.

7. A pharmaceutical composition comprising a compound according to any of claims 1 to 4 together with one or more other therapeutic agents, preferably selected from the group consisting of (1) a corticosteroid, for example fluticasone or budesonide; (2) a β2-adrenoreceptor agonist, for example salmeterol or formeterol; (3) a leukotriene modulator, for example montelukast or pranlukast; (4) anticholinergic agents, for example selective muscarinic-3 (M3) receptor antagonists such as tiotropium bromide; (5) phosphodiesterase-IV (PDE-IV) inhibitors, for example roflumilast or cilomilast; (6) an antitussive agent, such as codeine or dextramorphan; (7) a non-steroidal anti-inflammatory agent (NSAID), for example ibuprofen or ketoprofen and (8) an H1 antagonist or inverse agonist, for example loratidine or cetirizine.

8. A method of preventing, treating or ameliorating a disease mediated by the H4 receptor alone or by the H1 and H4 receptors in combination in a subject in need thereof comprising the administration of a therapeutically effective amount of a compound according to any of claims 1 to 4.

9. A method according to Claim 8 wherein the disease is mediated by the H4 receptor alone or by the H1 and H4 receptors in combination.

10. A method according to Claim 8 wherein the disease wherein the disease mediated by the H4 receptor alone or by the H1 and H4 receptors in combination is selected from: inflammatory diseases, asthma, psoriasis, rheumatoid arthritis, Crohn's disease, inflammatory bowel disease, ulcerative colitis, allergic rhinitis and other allergic diseases, and atopic dermatitis and other dermatological disorders.

11. Use of a compound according to any of claims 1 to 4 for the manufacture of a medicament useful for the prevention, treatment, or suppression of a disease mediated by the H4 receptor alone or by the H1 and H4 receptors in combination in a human subject in need thereof.
